Europäisches Patentamt

European Patent Office

Office européen des brevets

(19)

(11) Veröffentlichungsnummer: **0 161 204**

**A1**

(12) # EUROPÄISCHE PATENTANMELDUNG

(21) Anmeldenummer: 85730033.9

(22) Anmeldetag: 02.03.85

(51) Int. Cl.⁴: **A 61 M 35/00**

---

(30) Priorität: 02.03.84 DE 8406564 U
31.03.84 DE 8409955 U

(43) Veröffentlichungstag der Anmeldung·
13.11.85 Patentblatt 85/46

(84) Benannte Vertragsstaaten:
AT BE CH DE FR GB IT LI NL SE

(71) Anmelder: Meinass, Ulrich W.
Auf der Hude 85
D-2120 Lüneburg(DE)

(72) Erfinder: Meinass, Ulrich W.
Auf der Hude 85
D-2120 Lüneburg(DE)

(74) Vertreter: Scholz, Hartmut, Dipl.-Ing.
Wielandstrasse 18
D-1000 Berlin 41(DE)

---

(54) **Peloid-Heilpackung.**

(57) Heilpackung (4) mit einer heilwirksamen Peloidfüllung zur äußeren Auflage auf einen zu behandelnden Körperteil eines Patienten, dadurch gekennzeichnet, daß das Peloid (2) einem porösen Trägerkern (3) zugeordnet ist.

Fig. 2

EP 0 161 204 A1

Croydon Printing Company Ltd.

- 1 -

11-162/85-EP

Peloid-Heilpackung

Beschreibung

Die Erfindung betrifft eine Heilpackung mit einer heilwirksamen Peloidfüllung zur äußeren Auflage auf einen zu behandelnden Körperteil eines Patienten.

Zur physiotherapeutischen Behandlung in Massagepraxen, medizinischen Badebetrieben, Kliniken, Kur- und Krankenhäusern u.s.w. ist es allgemein bekannt, solche Peloid-Heilpackungen zu verwenden. Diese Peloid-Heilpackungen bestehen beispielsweise aus Fango, Fangoparaffin, Naturmoor, Moorparaffin, Heublumen, Schlick usw. Überwiegend handelt es sich dabei um wiederverwendbare Packungen, die an Ort und Stelle mit Hilfe aufwendiger Rührwerke und Aufbereitungsanlagen hergestellt und nach Gebrauch sterilisiert werden müssen.

Darüberhinaus gibt es Einweg-Heilpackungen, wie sie beispielsweise in der DE-PS 26 11 928 beschrieben sind, die aus einem Beutel mit einer Peloidfüllung bestehen. Diese Beutel halten die Peloidfüllung zusammen und bestehen auf einer Seite aus einem durchlässigen Vlies und auf der anderen Seite aus einer dichten Kunststoffolie. Diese Beutel haben jedoch den Nachteil, daß die Peloidfüllung leicht in ihnen verrutschen kann und dadurch an Körperrundungen nie gleichmäßig aufgetragen bleibt. Ferner ist das Vlies zwar wasserdurchlässig, aber es muß doch so dicht sein, daß es das Peloid während des Transports und der Lagerung fest zusammenhält. Ein direkter Kontakt des Peloids mit der behandelten Körperpartie wird durch ein derartiges Vlies aber be- oder verhindert.

Aufgabe der vorliegenden Erfindung ist es, eine Heilpackung der eingangs beschriebenen Art zu schaffen, mit der ein guter Kontakt des Peloids mit dem Körper und eine gleichbleibende Schichtdicke auf allen in Berührung stehenden Körperpartien gewährleistet wird.

Gelöst wird diese Aufgabe dadurch, daß das Peloid einem Trägerkern zugeordnet ist. Durch diese Maßnahmen wird eine Heilpackung geschaffen, in der das Peloid von einem Trägerkern in seiner Lage und Schichtdicke gehalten wird, wodurch die Peloidfüllung nicht mehr verrutschen kann. Ferner kann zum Abdecken ein großporiges Vlies verwendet werden, weil die Pe-

- 2 -

loidfüllung jetzt von dem Trägerkern zusammengehalten wird. Dadurch ist jetzt ein guter Kontakt des Peloids mit dem Körper möglich, oder es kann zur noch weiteren Verbesserung dieses Kontakts ganz auf eine Abdeckung verzichtet werden.

Weitere vorteilhafte Maßnahmen sind in den Unteransprüchen beschrieben. Die Erfindung ist in der beiliegenden Zeichnung dargestellt und wird nachfolgend näher beschrieben; es zeigt:

Fig. 1   die Schrägdraufsicht auf eine Heilpackung mit Trägerkern und Folie;

Fig. 2   den Schnitt durch eine Heilpackung nach der Fig. 1;

Fig. 3   die Schrägdraufsicht auf eine Heilpackung aus einen mit Peloid getränkten Trägerkern.

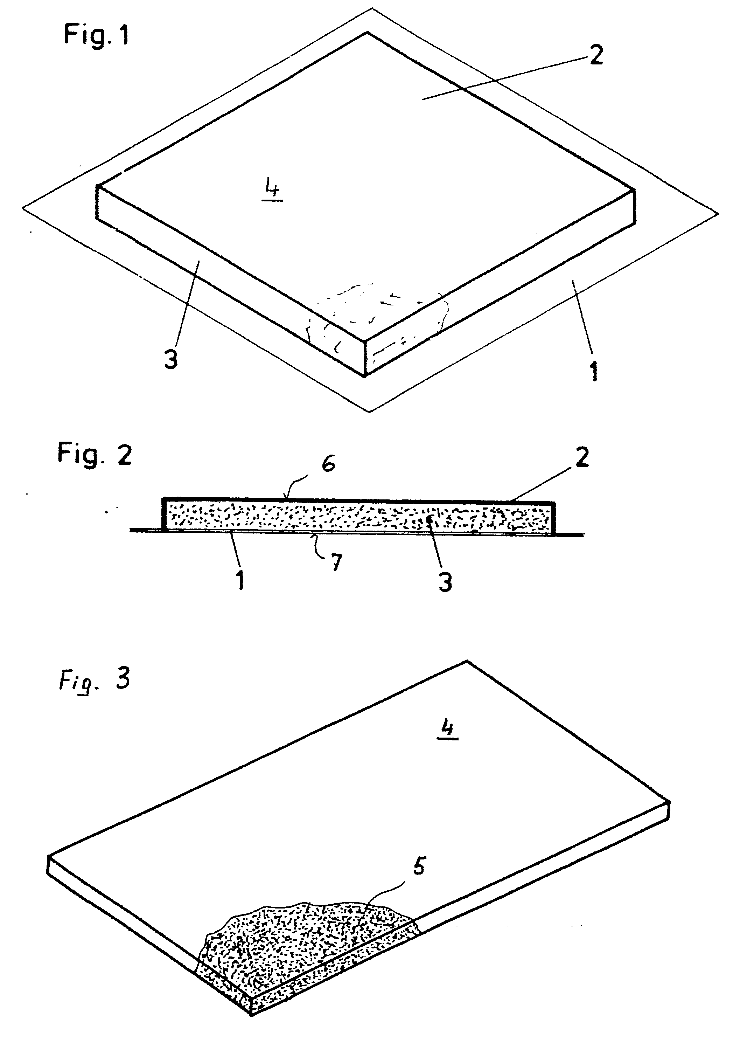

Die in den Figuren 1 und 2 dargestellte Heilpackung 4 ist an ihrer Oberseite 6  mit einer Folie 1 versehen, die mit einem Trägerkern 3 verbunden ist. Auf ihre Unterseite 7 ist, wie die Fig. 2 zeigt, eine dünne Schicht von Peloid 2 aufgetragen. Der Trägerkern 3 ist porös, und das Peloid 2 befindet sich auch in den Poren 5.

Die Heilpackung 4 kann kalt oder warm aufgelegt werden. Zur Erwärmung wird üblicherweise ein - nicht dargestelltes - Heizkissen verwendet. Die dichte Folie 1 vermeidet dabei eine Verschmutzung eines solchen Heizkissens.

Bei der in der Fig. 3 dargestellten Ausführung besteht die Heilpackung 4 lediglich aus einem mit Peloid 2 getränkten Trägerkern 3. Das Peloid 2 befindet sich dabei gänzlich in den Poren 5, die sowohl an der Oberseite 6 als auch an der Unterseite 7 als zellartige Struktur auftreten.

11-162/85-EP

Ansprüche

1. Heilpackung mit einer heilwirksamen Peloidfüllung zur äußeren Auflage auf einen zu behandelnden Körperteil eines Patienten, dadurch gekennzeichnet, daß das Peloid (2) einem Trägerkern (3) zugeordnet ist.

2. Heilpackung nach Anspruch 1, dadurch gekennzeichnet, daß der Trägerkern (3) porös ist.

3. Heilpackung nach den Ansprüchen 1 und 2, dadurch gekennzeichnet, daß der Trägerkern (3) an seiner Oberseite (6) mit dem Peloid (2) beschichtet ist.

4. Heilpackung nach den Ansprüchen 1 bis 3, dadurch gekennzeichnet, daß das der poröse Trägerkern (3) mit dem Peloid (2) getränkt ist.

5. Heilpackung nach den Ansprüchen 1 bis 4, dadurch gekennzeichnet, daß der poröse Trägerkern (3) an seiner nicht mit Peloid (2) beschichteten Unterseite (7) mit einer undurchlässigen Folie (1) versehen ist.

6. Heilpackung nach den Ansprüchen 1 und 5, dadurch gekennzeichnet, daß der Trägerkern (3) mit der Folie (1) verklebt ist.

7. Heilpackung nach den Ansprüchen 1 und 5, dadurch gekennzeichnet, daß der Trägerkern (3) aus flexiblem Material besteht.

8. Heilpackung nach den Ansprüchen 1 bis 6, dadurch gekennzeichnet, daß der poröse Trägerkern (3) aus Schaumstoff besteht.

9. Heilpackung nach den Ansprüchen 1 bis 8, dadurch gekennzeichnet, daß der poröse Trägerkern (3) an seiner Oberseite (6) und seiner Unterseite (7) eine zellartige Struktur aufweist.

Fig. 1

2

4

3

1

Fig. 2

6

2

1

7

3

Fig. 3

4

5

| | | | |
|---|---|---|---|

# Europäisches Patentamt

## EUROPÄISCHER RECHERCHENBERICHT

| EINSCHLÄGIGE DOKUMENTE | | | EP 85730033.9 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl 4)** |
| Y | DE - A1 - 3 207 899 (H.STULZ) | 1,2 | A 61 M 35/00 |
| A | * Gesamt * | 4-9 | |
| | -- | | |
| Y | DE - A - 1 566 322 (EIFELFANGO) | 1,2 | |
| | * Gesamt * | | |
| | -- | | |
| A | DE - A - 2 301 821 (G.HUBMANN) | | |
| | * Gesamt * | | |
| | -- | | |
| D,A | DE - C2 - 2 611 928 (P.HASLAUER) | 1 | |
| | * Gesamt * | | |
| | -- | | |
| A | WO - A1 - 81/03 283 (BAXTER TRAV. LAB.) | | **RECHERCHIERTE SACHGEBIETE (Int. Cl.4)** |
| | * Gesamt * | | |
| | ---- | | A 61 F 7/00 |
| | | | A 61 M 35/00 |

Der vorliegende Recherchenbericht wurde für alle Patentansprüche erstellt.

| Recherchenort | Abschlußdatum der Recherche | Prüfer |
|---|---|---|
| WIEN | 03-07-1985 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82